(19) **Europäisches Patentamt European Patent Office Office européen des brevets**

(11) **EP 4 052 733 B1**

(12) **EUROPEAN PATENT SPECIFICATION**

(45) Date of publication and mention of the grant of the patent:
**03.09.2025 Bulletin 2025/36**

(21) Application number: **22159197.7**

(22) Date of filing: **28.02.2022**

(51) International Patent Classification (IPC):
*A61L 2/24* (2006.01)   *A61L 2/10* (2006.01)
*G05D 1/00* (2024.01)   *G08C 17/02* (2006.01)
*H04Q 9/00* (2006.01)

(52) Cooperative Patent Classification (CPC):
**A61L 2/24; A61L 2/10; G05D 1/024; G05D 1/0272; G08C 17/02; H04Q 9/00;** A61L 2202/14; A61L 2202/16; A61L 2202/25

(54) **METHOD FOR CONTROLLING A MOBILE ROBOTIC APPARATUS FOR DISINFECTING A SPACE AND A MOBILE ROBOTIC APPARATUS FOR DISINFECTING A SPACE IMPLEMENTING SUCH METHOD**

VERFAHREN ZUR STEUERUNG EINER MOBILEN ROBOTERVORRICHTUNG ZUR DESINFEKTION EINES RAUMES UND MOBILE ROBOTERVORRICHTUNG ZUR DESINFEKTION EINES RAUMES MIT DEM VERFAHREN

PROCÉDÉ DE COMMANDE D'UN APPAREIL ROBOTIQUE MOBILE POUR DÉSINFECTER UN ESPACE ET APPAREIL ROBOTIQUE MOBILE POUR DÉSINFECTER UN ESPACE METTANT EN UVRE UN TEL PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2021 IT 202100004727**

(43) Date of publication of application:
**07.09.2022 Bulletin 2022/36**

(73) Proprietor: **Next Generation Robotics S.r.l.**
**56123 Pisa (IT)**

(72) Inventors:
• **FRISOLI, Antonio**
**I-56010 San Giuliano Terme, Frazione Arena Metato,**
**PISA (IT)**
• **GABARDI, Massimiliano**
**I-39012 Merano, BOLZANO (IT)**
• **LEONARDIS, Daniele**
**I-70026 PISA (IT)**
• **CHIARADIA, Domenico**
**I-56121 PISA (IT)**
• **TISENI, Luca**
**I-62017 Porto Recanati, MACERATA (IT)**
• **SOLAZZI, Massimiliano**
**I-56023 Cascina, PISA (IT)**

(74) Representative: **Mozzi, Matteo et al**
**Jacobacci & Partners S.p.A.**
**Via Senato, 8**
**20121 Milano (IT)**

(56) References cited:
EP-A1- 3 033 115     WO-A1-2016/077403
WO-A1-2019/190967     WO-A1-2023/003474
US-A1- 2012 223 216     US-A1- 2020 179 543

## Description

Field of the invention

The present invention relates to the field of robotics, and in particular, to a method for controlling a mobile robotic apparatus for disinfecting a space and to a mobile robotic apparatus for disinfecting a space implementing such method.

Technological background of the invention

The spread of the SARS-CoV-2 virus has posed new challenges and constraints to daily life, particularly concerning safe access to public spaces, shared spaces, and workplaces.

In this regard, robotic technology, along with artificial intelligence and automation, can be used effectively to disinfect spaces, administer drugs or serve food, and perform remote diagnostics, limiting human operators' risk of exposure to potentially contaminated spaces.

As for disinfection, there are several so-called "no-touch" solutions for disinfecting spaces and surfaces, where the use of C-type ultraviolet light radiation or simply UV-C radiation is very effective for inactivating viruses and disinfecting bacteria on surfaces, reducing contamination on high-contact surfaces.

The evolution of robotic technology, combined with UV-C radiation systems, has led to the development of mobile robotic apparatus with UV-C radiation sources which represent a significant advantage over fixed UV-C radiation sources.

Indeed, a mobile UV-C radiation source can overcome many of the obvious limitations of a fixed UV-C radiation source which, for example, cannot provide equivalent levels of UV-C radiation dose at different distances from the source, considering that an administered dose of ultraviolet radiation is a function of both the intensity of the UV-C radiation and the exposure time to the UV-C radiation.

Nowadays, the need is strongly felt to control a mobile robotic apparatus for disinfecting a space with a source of C-type ultraviolet light (or Ultraviolet-C, UV-C) radiation so as to optimize as much as possible both the path to be followed within the space to be disinfected and the provision of UV-C radiation such as to ensure an appropriate UV-C radiation dose on each contact surface in the space.

WO 2016/077403 A1 and EP 3 033 115 A1 deal with robotic disinfecting apparatuses.

Summary

It is an object of the present invention to devise and provide a method for controlling a mobile robotic apparatus for disinfecting a space which allows obviating at least partially the drawbacks described above with reference to the prior art, in particular, which allows optimizing as much as possible both the path to be followed within the space to be disinfected and the provision of a C-type ultraviolet light (ultraviolet-C, UV-C) radiation such as to ensure a respective appropriate UV-C radiation dose on each contact surface to be disinfected within the space.

Such an object is achieved by a method according to claim 1.

Preferred embodiments of said method are defined in the dependent claims.

The present invention further relates to a mobile robotic apparatus for disinfecting a space implementing such a method.

Brief description of the drawings

Further features and advantages of the method and the mobile robotic apparatus according to the invention will be apparent from the following description which illustrates preferred embodiments, given by way of indicative, non-limiting examples, with reference to the accompanying figures, in which:

- figures 1a and 1b show, respectively, a perspective view from above and a side perspective view of a mobile robotic apparatus for disinfecting a space according to an embodiment of the present invention;
- figure 2 shows a front view of a mobile robotic apparatus for disinfecting a space according to a further embodiment of the present invention;
- figure 3 shows, by means of a block chart, an architecture for controlling a mobile robotic apparatus for disinfecting a space according to an embodiment of the present invention;

- figure 4a diagrammatically shows a space in which it is represented an example of a path trajectory which can be traveled through by a mobile robotic apparatus for disinfecting a space according to the present invention;
- figure 4b diagrammatically shows the space in figure 4a in which it is represented a further example of a path trajectory which can be traveled through by a mobile robotic apparatus for disinfecting a space according to the present invention;

- figures 5a-5f diagrammatically shows a space in which it is represented in successive instants of time a further example of a path trajectory which can be traveled through by a mobile robotic apparatus for disinfecting a space according to the present invention, and
- figure 6 shows, by means of a block chart, a method for controlling a mobile robotic apparatus for disinfecting a space, according to an embodiment of the present invention.

It is worth noting that, in the aforesaid figures, equivalent or similar elements are indicated by the same numeric and/or alphanumeric references.

Detailed description

A mobile robotic apparatus for disinfecting a space will now be described with reference to the aforesaid figures.

The mobile robotic apparatus for disinfecting a space, hereinafter also mobile robotic apparatus or simply apparatus, is indicated as a whole by reference numeral 1.

The space to be disinfected is indicated in the figures by reference numeral 100 instead.

For the purposes of this description, "space" means any confined space, either indoors or outdoors, which requires disinfection periodically and/or after use to safeguard the health and safety of the users of the space.

Examples of "spaces" are meeting rooms, conference rooms, operating rooms, medical examination rooms, airport or train station lobbies, waiting rooms, rooms, bars, restaurants, classrooms, gymnasiums, and so on.

The space 100 comprises a plurality of contact surfaces to be disinfected present within the space 100.

"Contact surface" means any surface that can be touched by a user.

Examples of contact surfaces to be disinfected are door and/or window handles, furniture knobs, the top of a table or furniture in general, comprising objects resting thereon, the back and/or arms of a chair, the legs of a table or furniture in general, a shelf, a wall, but also office equipment such as keyboards, mouse, screens, computers, and so on.

Each contact surface to be disinfected is associated with a criticality level with respect to which a related level of disinfection is to be ensured.

Each criticality level is associated with the contact frequency and thus the risk that such a surface may be exposed to a virus or other contaminant.

The criticality level is determined according to the type of contact surface to be disinfected.

For example, a door handle of an access door to the space, frequently in contact with the hands of the users for opening and closing the door, will have a set criticality level to which a respective level of disinfection corresponds, greater than the set criticality level to which a respective level of disinfection corresponds of a shelf present inside the space, less frequently in contact with the hands of the users.

Referring back to the apparatus 1, the apparatus 1 comprises a mobile base 2 comprising a plurality of wheels 3, preferably omnidirectional wheels.

The apparatus 1 further comprises at least one C-type ultraviolet light radiation source 4 - or simply at least one Ultraviolet-C, UV-C, radiation source 4 - mounted on the mobile base 2.

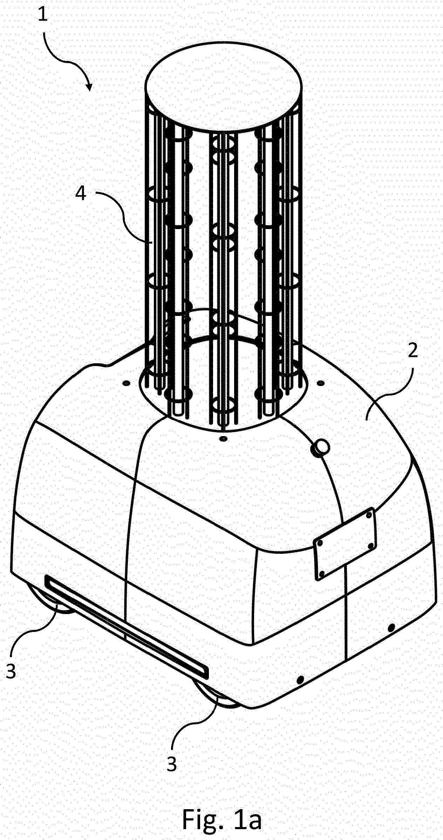

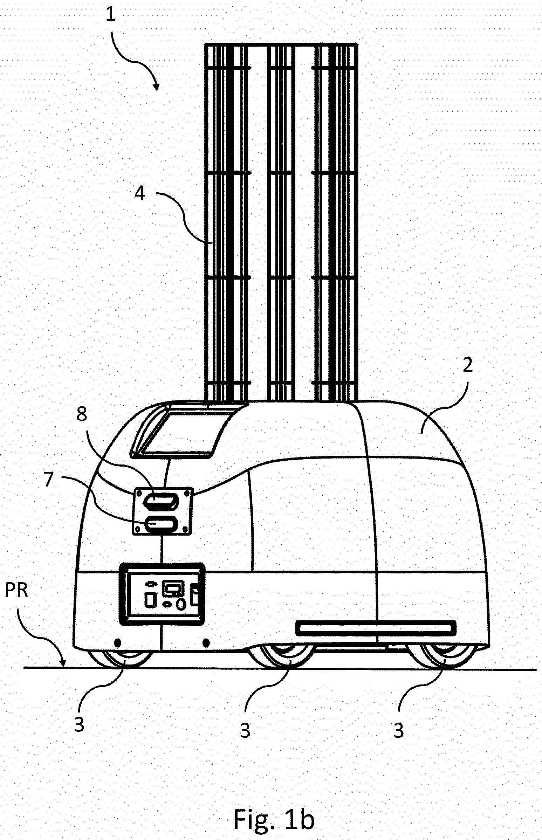

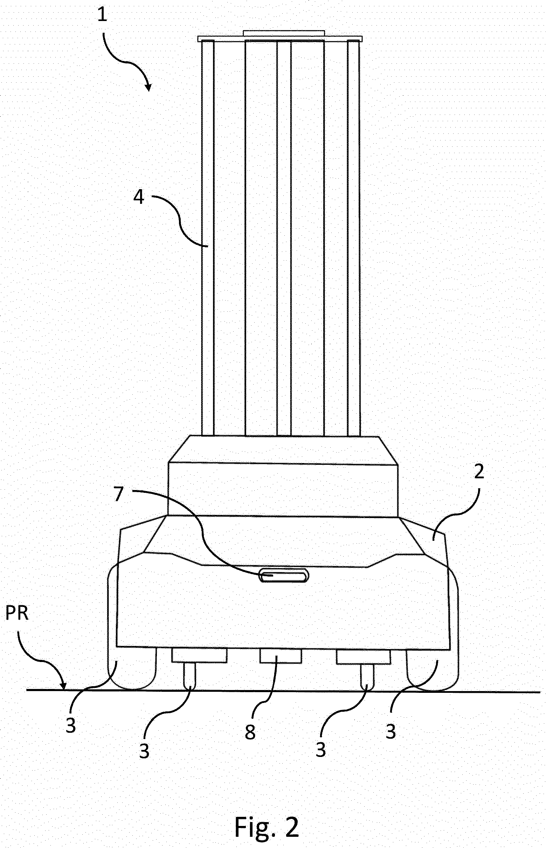

According to an embodiment shown in figures 1a, 1b and 2, the at least one C-type ultraviolet light radiation source 4 comprises a plurality of C-type ultraviolet light radiation lamps.

In greater detail, each lamp has a tubular shape and extends vertically from the mobile base 2 along a direction substantially orthogonal to a reference plane PR, i.e., a plane of motion on which the apparatus 1 can move (diagrammatically shown in figures 1b and 2).

The plurality of C-type ultraviolet light radiation lamps is mounted to the mobile base 2 at installation points distributed on the mobile base 2, such as in the center, along a circumference, and preferably equidistant from each other.

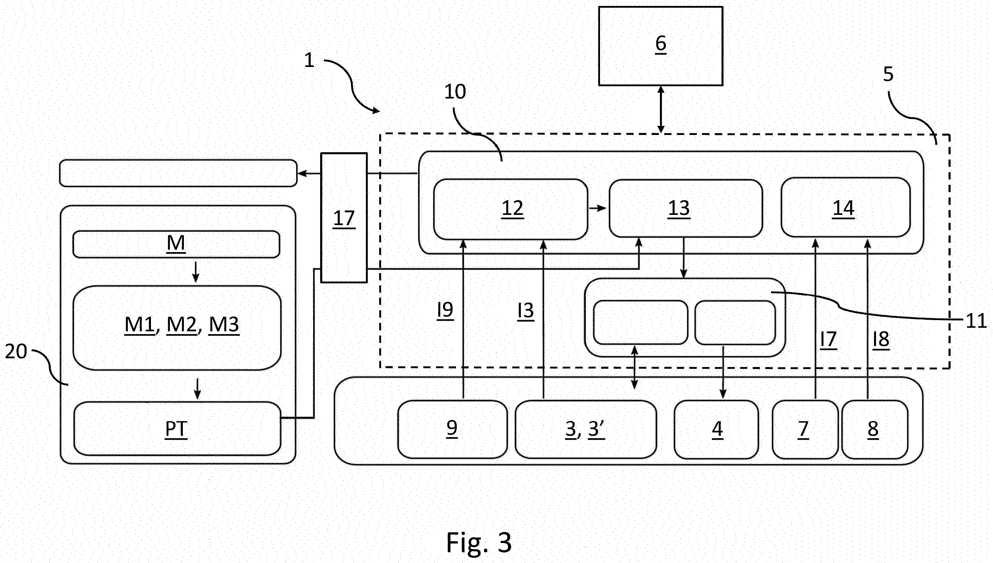

The apparatus 1, in general, further comprises a data processing unit 5, shown in figure 3, operatively connected to the at least one C-type ultraviolet light radiation source 4.

The data processing unit 5 is integrated into the mobile base 2.

The data processing unit 5 is, for example, one or more microcontrollers or microprocessors.

From a functional point of view, as will be described with particular reference to the architecture in figure 3, the data processing unit 5 comprises hardware modules appropriately configured from a software point of view and/or software logic for controlling the operation of the apparatus 1.

The apparatus 1 further comprises a memory unit 6 operatively connected to the data processing unit 5.

The memory unit 6, also integrated into mobile base 2, can be either internal or external (e.g., as shown in figure 3) to the data processing unit 5.

It is worth noting that the memory unit 6 is configured to store one or more program codes which are executable by the data processing unit 5 and data generated and processed following the execution of one or more program codes by the data processing unit 5.

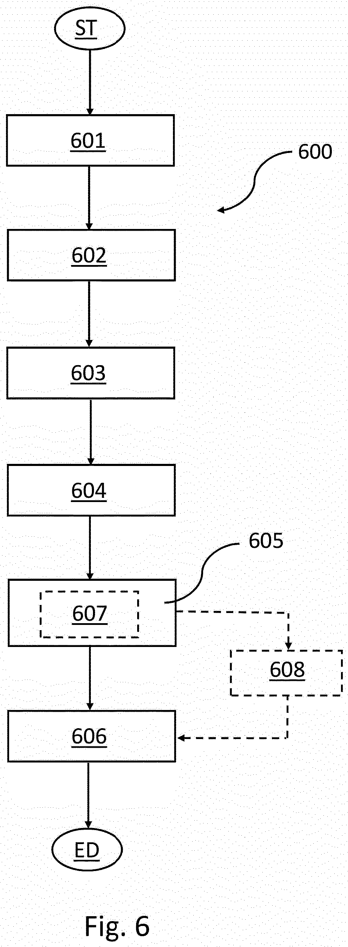

In this regard, as will also be reiterated below, the data processing unit 5 is configured to perform a method for controlling the mobile robotic apparatus 1, described later with particular reference also to figure 6.

The apparatus 1 further comprises at least one first artificial vision sensor 7 operatively connected to the data processing unit 5.

The at least one first artificial vision sensor 7 is configured to provide the data processing unit 5 with first information I7 representative of a geometry of the space 100 explored by the apparatus 1.

Examples of "first information I7" are RGB images and point clouds (RGB+Depth) related to the explored space.

The at least one first artificial vision sensor 7 is preferably positioned at the front of the mobile base 2, as shown for example in figures 1b and 2.

Examples of the at least one first artificial vision sensor 7 are a three-dimensional depth camera, light detection and ranging (LIDAR) sensors, ultrasonic sensors, sensorized mechanical buffers, and in general any sensor which allows acquiring data about the positioning of a point in the space with respect to the mobile robotic apparatus, which can be used either together or individually.

According to a further embodiment, in combination with the preceding embodiment, the apparatus 1 further comprises at least one second artificial vision sensor 8 configured to provide the data processing unit 5 with second information I8 representative of the space 100 explored by the apparatus 1.

Examples of "second information I8" are RGB images and point clouds (RGB+Depth) related to the explored space.

The at least one second artificial vision sensor 8 is preferably positioned at the front of the mobile base 2, as shown for example in figure 2.

Examples of the at least one second artificial vision sensor 8 is a depth detection module (LIDAR) configured to determine the distance of an object (obstacle) or a surface from the apparatus 1, a three-dimensional depth camera, ultrasonic sensors, sensorized mechanical buffers, and generally any sensor which allows the acquisition of data about the positioning of a point in the space with respect to the mobile robotic apparatus, which may be used either together or individually.

The apparatus 1 further comprises at least one inertial measurement unit (IMU) 9, operatively connected to the data processing unit 5.

The at least one inertial measurement unit 9, integrated into the mobile base 2, is configured to provide the data processing unit 5 with information I9 representative of a dynamics (e.g., linear accelerations, velocities and angular accelerations, orientation of the earth's magnetic field with respect to the sensor, and orientation of gravity with respect to the sensor) of the apparatus 1 during its movement.

The inertial measurement unit or IMU 9 is, for example, a multiple sensor including within it at least one accelerometer, at least one magnetometer, and at least one gyroscope, which allow extracting the information representative of the above listed dynamics of the apparatus 1 during its movement.

The apparatus 1 further comprises a plurality of odometer sensors 3' configured to provide the data processing unit 5 with information I3 representative of a displacement of the robotic apparatus 1 within the space 100.

Each of the odometer sensors of the plurality of odometer sensors 3' is operatively connected to a respective wheel of said plurality of wheels 3.

Each odometer sensor 3' is, for example, an angular position transducer (encoder) configured to provide an information I3 representative of an angular displacement of the respective wheel 3 to which the odometer sensor is operatively connected.

With particular reference to figure 3, it should be noted that, from a functional point of view, the data processing unit 5 comprises a first control module 10 and a second control module 11.

The first control module 10 may be referred to as a high-level controller of the apparatus 1 while the second control module 11 may be referred to as a low-level controller of the apparatus 1.

The first control module 10 comprises an odometer module 12 configured to determine the position of the mobile robotic apparatus 1 in the space 100 based on the information I9 representative of the dynamics of the apparatus 1 during its movement provided by the at least one inertial measurement unit 9 and the information I3 representative of a displacement of the robotic apparatus 1 within the space 100 provided by the plurality of odometer sensors 3'.

The first control module 10 further comprises a position and orientation control module 13 of the apparatus 1 configured to determine control signals SC to be provided to the apparatus 1 based on the position of the mobile robotic apparatus 1 in the space 100 determined by the odometer module 11 and an optimized reference path trajectory PT provided by a remote computer 20, described in greater detail hereinafter.

The first control module 10 further comprises a simultaneous localization and mapping module (SLAM) 14 configured to determine the localization of the apparatus 1 and the simultaneous mapping of the space 100 in which the apparatus 1 is located, based on the first information I7 representative of a geometry of the space 100 explorable by the apparatus 1 and the second information I8 representative of a geometry of the space 100 explorable by the apparatus 1 provided by the at least one first artificial vision sensor 7 and the at least one second artificial vision sensor 8, respectively.

The localization of the apparatus 1 and the simultaneous mapping of the space 100 within which the apparatus 1 is located are both used in real-time for navigation control of the apparatus 1.

Furthermore, as will be reiterated below, the simultaneous mapping of the space 100 in which the apparatus 1 is located, once acquired, is also used as input into a trajectory optimization algorithm, which allows to generate the optimal sanitization trajectories.

The second control module 11 comprises a wheel speed control module 15 configured to control the plurality of omnidirectional wheels 3 of the apparatus 1 based on the control signals SC provided by the position and orientation control module 13 of the apparatus 1 and based on the information I3 representative of a displacement of the robotic apparatus 1 within the space 100 provided by the plurality of odometer sensors 3'.

Examples of control signals SC that may be sent to the wheel speed control module 15 are linear and/or angular velocity references, or position and/or orientation references.

The second control module 11 further comprises a C-type ultraviolet light radiation source control module 16 configured to control the at least one C-type ultraviolet light radiation source 4 based on the control signals SC provided by the position and orientation control module 13 of the apparatus 1.

A control signal SC which may be sent to the source control module 16 is, for example, an on/off digital signal.

Referring again to figure 3, according to a further embodiment, the apparatus 1 comprises a data communication module 17 operatively connected to the data processing unit 5.

The data communication module 17 is configured to be operatively connected to a remote computer 20, previously introduced, through a data communication network (not shown in the figures).

The remote computer 20, e.g., an electronic calculator or a personal computer, is configured to remotely control the operation of the mobile robotic apparatus 1.

In greater detail, the remote computer 20 is configured to receive information I5 representative of the operation of the apparatus 1 from the data processing unit 5.

In this manner, by means of the remote computer 20, it is possible for a user, to remotely supervise the behavior of the apparatus 1, in total safety with respect to the space 100 to be disinfected in which the apparatus 1 is located.

The remote computer 20 is also configured to perform an offline optimization of the control of the apparatus 1.

In greater detail, the remote computer 20 is configured to determine an optimized reference path trajectory PT from a static map M of the space 100 to be disinfected, employing a trajectory generation and movement planning algorithm specific to disinfection procedures based on the following three modules:

- a first module M1 of artificial potential field (APF) configured to determine the speed of the apparatus 1 and guide the apparatus 1 along the contact surfaces to be disinfected based on the distance therefrom and the amount of energy stored by the surfaces;
- a second module M2 of physical simulation of the radiation physics and the movement of the apparatus 1 in the space 100 configured to modulate over time the APF value and the amount of energy released on the surfaces;
- a third module M3 of automatic optimization configured to optimize the process based on a genetic algorithm (GA).

The remote computer 20 is also configured to provide the optimized reference path trajectory PT determined to the data processing unit 5 of the apparatus 1.

Returning generally to the mobile robotic apparatus 1, according to the present invention, the data processing unit 5 of the apparatus 1 is configured to acquire a map M of a space 100 to be disinfected.

Furthermore, the data processing unit 5 of the apparatus 1 is configured to acquire information of a plurality of contact surfaces to be disinfected present within the space 100 to be disinfected.

As mentioned earlier, each contact surface is associated with a set criticality level with respect to which a related level of disinfection is to be ensured.

The data processing unit 5 of the apparatus 1 is configured to determine an amount of C-type ultraviolet light radiation energy to be deposited, through the at least one C-type ultraviolet light radiation source 4 mounted on the mobile base 2 of the apparatus 1, on a respective contact surface of said plurality of contact surfaces to be disinfected.

The data processing unit 5 of the apparatus 1 is configured to determine the amount of UV-C radiation energy as a function of the criticality level of the contact surface to be disinfected, of the distance and the orientation of the contact surface to be disinfected with respect to the at least one UV-C radiation source 4, and of set operating features of the at least one UV-C radiation source 4.

The set operating features of the at least one UV-C radiation source 4 comprise: surface, geometric arrangement, shape, radiation power.

In order to determine the amount of UV-C radiation energy, the data processing unit 5 of the apparatus 1 is configured to implement a respective physical model of light radiation which can be represented by the following mathematical relationship (1):

$$I(\vec{r}, \hat{n}) = \frac{P}{4\pi A} \oiint_A \frac{\eta\,(\vec{r}, \hat{n})}{|\vec{r}|^2}\, dA \qquad (1)$$

where:

I = intensity (amount) of light radiation;
r = distance of the at least one UV-C radiation source 4 from the contact surface to be disinfected;
A = area of at least one UV-C radiation source 4;
n = normal of the contact surface to be disinfected;
$\eta$ = optical efficiency value, which can be determined using the following mathematical relationship (2):

$$\eta\,(\vec{r}, \hat{n}) = \begin{cases} \dfrac{|\vec{r} \cdot \hat{n}|}{|\vec{r}|} & \vec{r} \cdot \hat{n} \le 0 \\[2mm] 0 & \vec{r} \cdot \hat{n} > 0 \end{cases} \qquad (2)$$

Furthermore, the data processing unit 5 of the apparatus 1 is configured to determine, for each time instant $t_i$, $1 < i < N$, of a plurality of time instants, a respective virtual potential of attraction of the at least one UV-C radiation source 4 towards each contact surface to be disinfected of said plurality of contact surfaces to be disinfected, by modulating over time the virtual attraction potential of the at least one UV-C radiation source 4 towards each contact surface of said plurality of contact surfaces to be disinfected as a function of the amount of UV-C radiation energy determined for each contact surface to be disinfected and radiated at the previous time instant $t_{i-1}$.

The virtual (or artificial) potential of attraction is a value of energy level determinable by means of a mathematical model representative of a law of attraction or repulsion towards each contact surface to be disinfected of said plurality of contact surfaces to be disinfected.

The virtual (or artificial) potential of attraction can be determined, as an example, by implementing the following mathematical relationship (3):

$$U(\vec{r_i}, t) = -k \sum_i w_i(t) \frac{1}{|\vec{r_i}|^n} \qquad (3)$$

where:

U = virtual (or artificial) potential of attraction;
k = constant of determination of an intensity of the virtual potential;
n = exponent of determination of a change in the virtual potential as a function of the distance of the at least one UV-C radiation source 4 from a contact surface to be disinfected;
$w_i$ = weight of the contact surface to be disinfected determinable by the following mathematical relationship (4):

$$w_i(t) = \begin{cases} 1 + mf(\lambda_i) & \lambda_i < 1 \\ 0 & \lambda_i \ge 1 \end{cases} \qquad (4)$$

where:

$E_0$ = radiation energy density to be achieved to have a level of disinfection such that a specific virus or bacterium is inactivated;
$E_i$ = energy stored by the same contact surface to be disinfected in the previous transit of the apparatus 1;
$\lambda_i = \dfrac{E_i(t)}{E_0}$   variable function of the energies $E_i$ and $E_0$;
$f(\lambda_i)$ is a function of $\lambda_i$ defined positive i.e., such that $f(\lambda_i) \ge 0$ valid for every $\lambda_i \ge 0$
m = coefficient representing the maximum possible limit of the weight of the contact surface to be disinfected.

Therefore, the determined virtual potential of attraction of the at least one UV-C radiation source 4 towards a contact surface to be disinfected is function of both the radiation energy density to be achieved to have a level of disinfection such that a specific virus or bacterium is inactivated and the energy stored by the same contact surface to be disinfected in the previous transit of the apparatus 1.

The data processing unit 5 of the apparatus 1 is also configured to generate a path trajectory as a function of each determined virtual potential of attraction of the at least one UV-C radiation source 4 towards each contact surface to be disinfected.

It is worth noting that the sum of all potential contributions is a potential function which is then used to obtain by mathematical calculations a speed or acceleration to be given to the apparatus 1, either in real-time or in simulation.

Therefore, the path trajectory is generated as directional speed or acceleration to be provided to the apparatus 1, either in real-time or in simulation, on the basis of each determined virtual potential of attraction of the at least one UV-C radiation source 4 towards each contact surface to be disinfected, i.e. also of the energy stored by each contact surface to be disinfected in the previous transit of the apparatus 1.

In greater detail, the path trajectory (directional speed) is generated as gradient of the determined virtual potential of attraction of the at least one UV-C radiation source 4 towards each contact surface to be disinfected.

Finally, the data processing unit 5 is configured to control the apparatus 1 within the space 100 to be disinfected along the generated path trajectory.

According to an embodiment, in combination with the preceding one, the data processing unit 5 of the apparatus 1 is configured to generate the path trajectory based on a gradient of the determined virtual potential of attraction of the at least one UV-C radiation source 4 towards each contact surface to be disinfected to obtain a sliding of the mobile robotic apparatus 1 along obstacles encountered within the space to be disinfected along the path trajectory taken.

The gradient of the determined virtual potential can be expressed by the following mathematical relationship (5):

$$\vec{v} = \sum_i -\nabla U_i = \sum_i k\, n\, w_i(t)\, \frac{\vec{r_i}}{|\vec{r_i}|^{-(n+2)}} \qquad (5)$$

According to a further embodiment, in combination with any one of those described above, the data processing unit 5 of the apparatus 1 is configured to perform an optimization procedure of a plurality of parameters employed to determine a respective virtual potential of attraction of the at least one UV-C radiation source towards each contact surface to be disinfected of said plurality of contact surfaces to be disinfected, based on different values of virtual potential of attraction of the at least one UV-C radiation source towards each contact surface of said plurality of contact surfaces.

The optimized parameters introduced above with the references k, n, and m in the mathematical relationships (3) and (4) are representative of path trajectories capable of ensuring that the mobile robotic apparatus travels in a set minimum time through the space to be disinfected providing an appropriate radiation dose on each contact surface to be disinfected, i.e., travels an optimal sanitization trajectory.

As mentioned above, the optimization procedure may also employ as input the simultaneous mapping of the space 100 within which the apparatus 1 is located, once acquired.

According to an embodiment, in combination with any one of those described above, the acquisition of a map of a space 100 to be disinfected can be directly performed by the data processing unit 5 of the apparatus 1 or provided by the remote computer 20 with respect to the apparatus 1 and in communication therewith through the data communication network.

According to an embodiment, alternative to the preceding one, the acquisition of information of a plurality of contact surfaces to be disinfected can be performed by the data processing unit 5 of the apparatus 1 or provided by the remote computer 20 with respect to the apparatus 1 and in communication therewith through the data communication network.

With reference now also to figure 6, a method 600 for controlling a mobile robotic apparatus 1 for disinfecting a space 100, hereinafter also only method for controlling or simply method, according to the present invention, will be described.

It is worth noting that the components of apparatus 1 and the information previously described with reference to the apparatus 1 that will also be mentioned with reference to the method 600 will not be repeated in detail for the sake of brevity.

The method 600 comprises a symbolic step of starting ST.

The method 600 comprises a step a) of acquiring 601, by the data processing unit 5 of the mobile robotic apparatus 1, a map M of a space 100 to be disinfected.

The method 600 further comprises a step of b) acquiring 602, by the data processing unit 5 of the mobile robotic apparatus 1, information about a plurality of contact surfaces to be disinfected present within the space 100 to be disinfected.

Each contact surface to be disinfected is associated with a criticality level with respect to which a related level of disinfection is to be ensured.

The criticality level and the related level of disinfection have been defined above.

Examples of space 100 and contact surfaces to be disinfected have been provided above.

The method 600 further comprises a step of c) determining 603, by the data processing unit 5 of the mobile robotic apparatus 1, an amount of C-type ultraviolet light radiation energy to be deposited, by means of at least one ultraviolet-C, UV-C, radiation source 4 mounted on the mobile robotic apparatus 1, on a respective contact surface of said plurality of contact surfaces to be disinfected.

The amount of UV-C radiation energy is determined as a function of the criticality level of the contact surface, of the distance and the orientation of the contact surface to be disinfected with respect to the at least one UV-C radiation source 4, of set operating features of the at least one UV-C radiation source 4.

The set operating features of the at least one UV-C radiation source 4 comprise: surface, geometric arrangement, shape, radiation power.

The physical radiation model used to determine the amount of UV-C radiation energy was described above.

Again with reference to figure 6, the method 600 further comprises a step of d) determining 604, for each time instant $t_i$, $1 < i < N$, of a plurality of time instants, by the data processing unit 5 of the mobile robotic apparatus 1, a respective virtual potential of attraction of the at least UV-C radiation source 4 towards each contact surface of said plurality of contact surfaces, by modulating over time the virtual potential of attraction of the at least one UV-C radiation source 4 towards each contact surface to be disinfected of said plurality of contact surfaces as a function of the amount of radiation energy determined for each contact surface to be disinfected and radiated at the previous time instant $t_{i-1}$.

As previously described, the virtual (or artificial) potential of attraction is a value of energy level determinable by means of a mathematical model representative of a law of attraction or repulsion towards each contact surface to be disinfected of said plurality of contact surfaces to be disinfected.

The determined virtual potential of attraction of the at least one UV-C radiation source 4 towards a contact surface to be disinfected is function of both the radiation energy density to be achieved to have a level of disinfection such that a specific virus or bacterium is inactivated and the energy stored by the same contact surface to be disinfected in the previous transit of the apparatus 1.

The mathematical relationships underlying the determination of the virtual potential have been described above.

The method 600 further comprises a step e) of generating 605, by the data processing unit 5 of the mobile robotic apparatus 1, a path trajectory TP as a function of each determined virtual potential of attraction of the at least one UV-C radiation source 4 towards each contact surface to be disinfected.

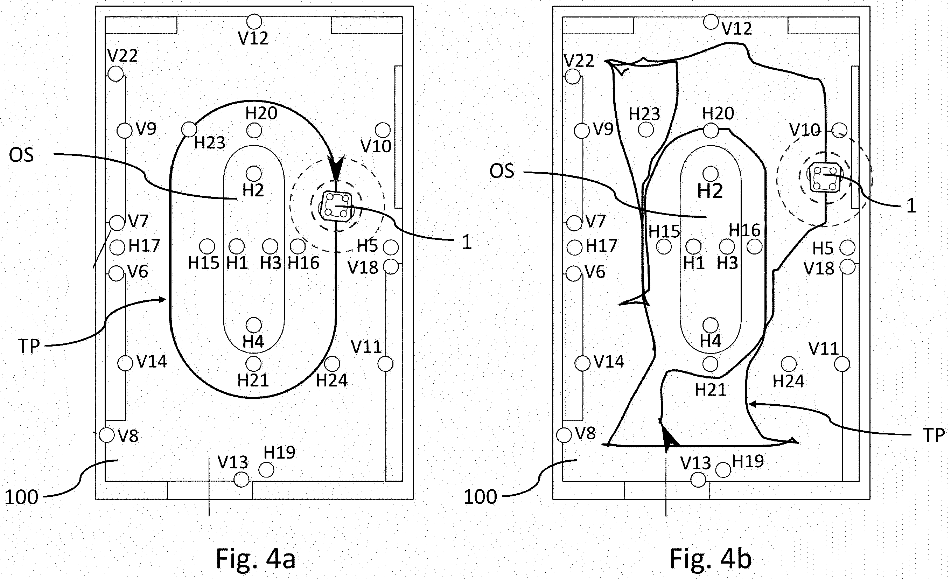

Figures 4a and 4b show a generated path trajectory TP which can be traveled by an apparatus 1 within a space 100 to be disinfected.

As previously described, the path trajectory is therefore generated as directional speed or acceleration to be provided to the apparatus 1, either in real-time or in simulation, on the basis of each determined virtual potential of attraction of the at least one UV-C radiation source 4 towards each contact surface to be disinfected, i.e. also of the energy stored by each contact surface to be disinfected in the previous transit of the mobile robotic apparatus 1.

In greater detail, the path trajectory (directional speed) is generated as gradient of the determined virtual potential of attraction of the at least one UV-C radiation source 4 towards each contact surface to be disinfected.

The method 600 further comprises a step f) of controlling 606, by the data processing unit 5 of the mobile robotic apparatus 1, the mobile robotic apparatus 1 along the generated path trajectory (figures 4a and 4b).

The method 600 comprises a symbolic step of ending ED.

According to an embodiment, in combination with the preceding one and shown with dashed lines in figure 6, the step of e) generating 605 further comprises a step of g) generating 607 the path trajectory based on a gradient of the determined virtual potential of attraction of the at least one UV-C radiation source 4 towards each contact surface to be disinfected to achieve a sliding of the mobile robotic apparatus 1 along obstacles encountered within the space to be disinfected along the traveled path trajectory.

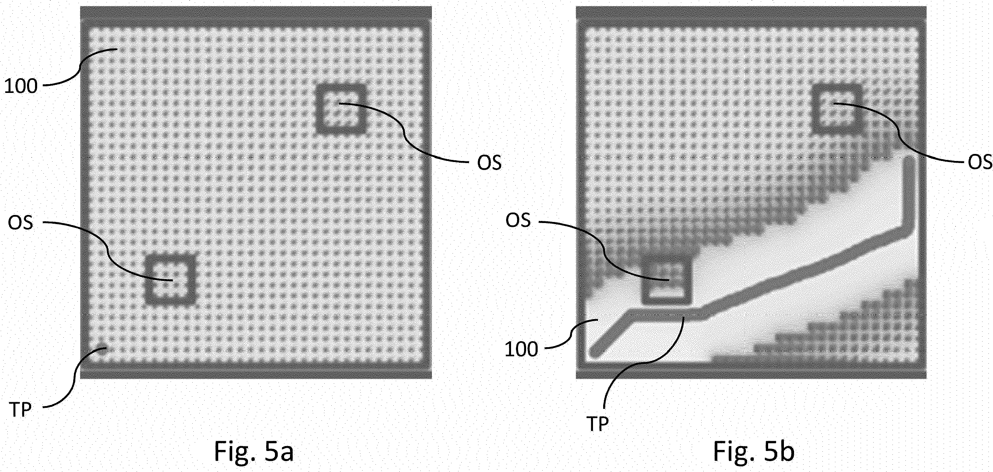

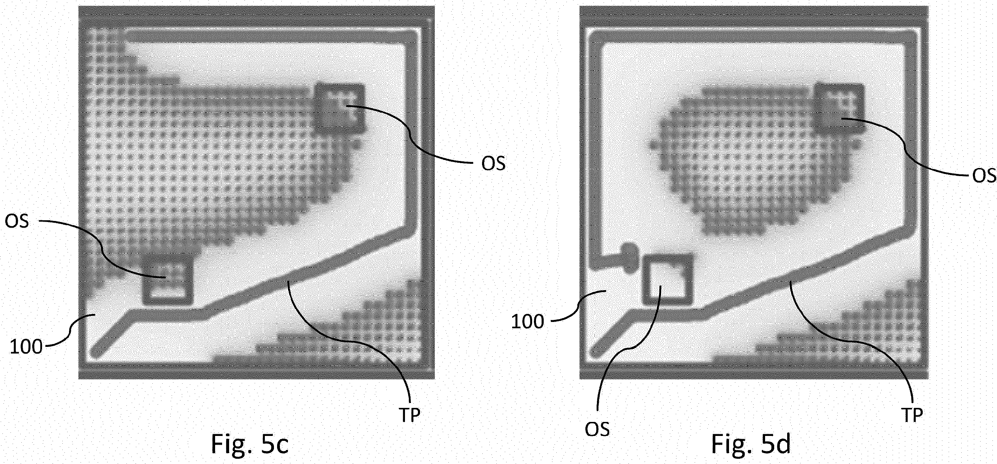

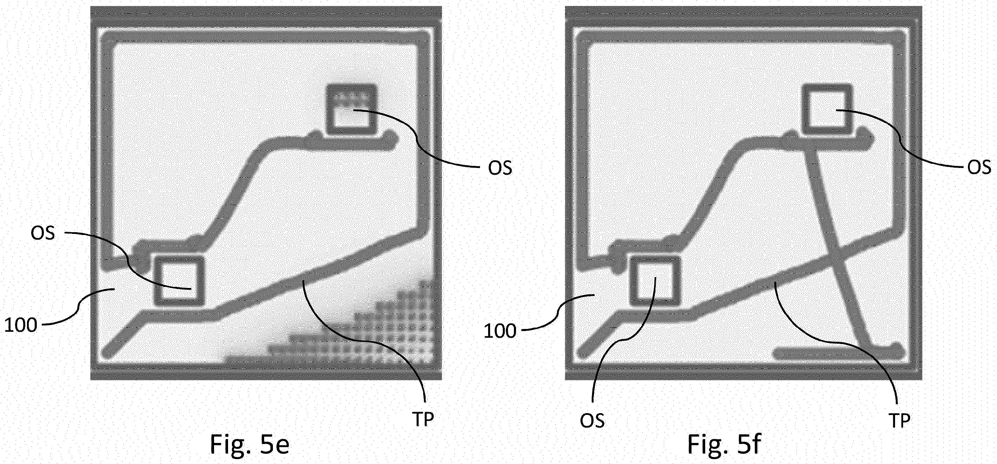

Examples of obstacles are shown in figures 4a, 4b, and 5a-5f, indicated by reference OS.

According to an embodiment, in combination with any one of those described above, the method 600 further comprises a step of h) performing 608 a procedure for optimizing a plurality of parameters employed to determine respective virtual potential of attraction of the at least one UV-C radiation source 4 towards each contact surface to be disinfected of said plurality of contact surfaces to be disinfected, based on different values of virtual potential of attraction of the at least one UV-C radiation source 4 towards each contact surface to be disinfected of said plurality of contact surfaces to be disinfected.

The optimized parameters introduced above with the references k, n, and m in the mathematical relations (3) and (4) are representative of optimized path trajectories capable of ensuring that the mobile robotic apparatus 1 travels in a set minimum time through the space 100 to be disinfected while providing an appropriate radiation dose for disinfection on each contact surface to be disinfected.

Figure 4b shows an optimized path trajectory TP which can be traveled by an apparatus 1 within a space 100 to be disinfected.

According to an embodiment, in combination with any one of those previously described, the step of a) acquiring 601 may be directly performed by the data processing unit 5 of the mobile robotic apparatus 1 or may be provided by a remote computer 20 with respect to the mobile robotic apparatus 1 and in communication therewith by means of a data communication network.

According to an embodiment, in combination with any one of those previously described, where the step of b) acquiring 602 may be directly performed by the data processing unit 5 of the mobile robotic apparatus 1 or may be provided by a remote computer 20 with respect to the mobile robotic apparatus 1 and communication therewith by means of a data communication network.

An example of operation of the mobile robotic apparatus 1 for disinfecting a space 100 to be disinfected will be described now with reference to the aforesaid figures.

A data processing unit 5 of a mobile robotic apparatus 1 acquires a map M of a space 100 to be disinfected.

The data processing unit 5 of the mobile robotic apparatus 1 acquires information of a plurality of contact surfaces to be disinfected present within the space 100 to be disinfected.

Each contact surface to be disinfected is associated with a criticality level with respect to which a related level of disinfection is to be ensured.

The data processing unit 5 of the mobile robotic apparatus 1 determines an amount of C-type ultraviolet light radiation energy to be deposited, by means of at least one UV-C radiation source 4 mounted on the mobile robotic apparatus 1, on a respective contact surface to be disinfected of said plurality of contact surfaces to be disinfected.

The amount of UV-C radiation energy is determined as a function of the criticality level of the contact surface, of the distance and the orientation of the contact surface to be disinfected with respect to the at least one UV-C radiation source 4, of set operating features of the at least one UV-C radiation source 4.

The data processing unit 5 of the mobile robotic apparatus 1 determines, for each time instant $t_i$, $1 < i < N$, of a plurality of time instants, a respective virtual potential of attraction of the at least one UV-C radiation source 4 towards each contact surface to be disinfected of said plurality of contact surfaces to be disinfected, by modulating over time the virtual potential of attraction of the at least one UV-C radiation source 4 towards each contact surface of said plurality of contact surfaces to be disinfected as a function of the amount of radiation energy determined for each contact surface to be disinfected and radiated at the previous time instant $t_{i-1}$.

The data processing unit 5 of the mobile robotic apparatus 1 generates a path trajectory TP as a function of each determined virtual potential of attraction of the at least one UV-C radiation source 4 towards each contact surface to be disinfected.

The data processing unit 5 of the mobile robotic apparatus 1 controls the mobile robotic apparatus 1 along the generated path trajectory TP.

As shown, the purpose of the present invention is fully achieved because the method and the mobile robotic apparatus have several advantages.

Indeed, the method according to the present invention allows obtaining a trajectory planning by employing a genetic algorithm which explores possible trajectories and disinfection outcomes of a mobile robotic apparatus moving in a tunable virtual (artificial) potential field and which is capable of maximizing the UV-C radiation dose delivered based on spatial geometry.

In particular, by comparing a conventional trajectory with an optimized trajectory, the method according to the present invention achieves better performance in terms of both coverage of radiated energy in the space and time required to complete the disinfection operation.

Furthermore, the fact that the method according to the present invention is based on an attractive potential field and on an iterative simulation based on radiation physics and optimization by genetic algorithm allows finding the most adaptive path trajectory to ensure completion of disinfection.

Furthermore, the fact that the method according to the present invention is able to simulate the path trajectory and the amount of C-type ultraviolet light radiation energy to be deposited, then stored in the previous transits of the apparatus, on the basis of the geometry of the space, advantageously allows to avoid a displacement within the space of sensors for detecting the deposited C-type ultraviolet light radiation.

Finally, the fact of being able to control the mobile robotic apparatus remotely, through the remote computer placed, for example, outside the space to be disinfected, advantageously allows increasing the safety of the user responsible of the supervision of disinfection operations.

Those skilled in the art may make changes and adaptations to the embodiments of the method and mobile robotic apparatus described above or can replace elements with others which are functionally equivalent in order to meet contingent needs.

**Claims**

1. A method (600) for controlling a mobile robotic apparatus (1) for disinfecting a space (100), comprising steps of:

   - a) acquiring (601), by a data processing unit (5) of a mobile robotic apparatus (1) for disinfecting a space (100), a map (M) of a space (100) to be disinfected;
   - b) acquiring (602), by the data processing unit (5) of the mobile robotic apparatus (1), information on a plurality of contact surfaces to be disinfected within the space (100) to be disinfected, each contact surface to be disinfected being associated with a criticality level with respect to which a related level of disinfection is to be ensured;
   - c) determining (603), by the data processing unit (5) of the mobile robotic apparatus (1), an amount of C-type ultraviolet light radiation energy to be deposited, by means of at least one ultraviolet-C, UV-C, radiation source (4) mounted to the mobile robotic apparatus (1), on a respective contact surface to be disinfected of said plurality of contact surfaces to be disinfected, said amount of UV-C radiation energy being determined as a function of the criticality level of the contact surface to be disinfected, of the distance and the orientation of the contact surface to be disinfected with respect to the at least one UV-C radiation source (4), of set operating features of the at least one UV-C radiation source (4);
   - d) determining (604), for each time instant $t_i$, $1 < i < N$, of a plurality of time instants, by the data processing unit (5) of the mobile robotic apparatus (1), a respective virtual potential of attraction of the at least one UV-C radiation source (4) towards each contact surface to be disinfected of said plurality of contact surfaces to be disinfected, by modulating over time the virtual potential of attraction of the at least one UV-C radiation source (4) towards each contact surface to be disinfected of said plurality of contact surfaces as a function of the amount of radiation energy determined for each contact surface to be disinfected and radiated at the previous time instant $t_{i-1}$, said virtual potential of attraction being a value of energy level determinable by means of a mathematical model representative of a law of attraction or repulsion towards each contact surface to be disinfected of said plurality of contact surfaces to be disinfected;
   - e) generating (605), by the data processing unit (5) of the mobile robotic apparatus (1), a path trajectory (TP) as a function of each determined virtual potential of attraction of the at least one UV-C radiation source (4) towards each contact surface to be disinfected, the path trajectory being generated as directional speed or acceleration to be provided to the mobile robotic apparatus (1), either in real-time or in simulation, on the basis of each determined virtual potential of attraction of the at least one UV-C radiation source (4) towards each contact surface to be disinfected, also comprising the energy stored by each contact surface to be disinfected in the previous transit of the mobile robotic apparatus (1);
   - f) controlling (606), by the data processing unit (5) of the mobile robotic apparatus (1), the mobile robotic apparatus (1) along the generated path trajectory (TP).

2. The method (600) according to claim 1, wherein the step e) further comprises a step of:

   - g) generating (607) the path trajectory (TP) based on a gradient of the determined virtual potential of attraction of the at least one UV-C radiation source (4) towards each contact surface to obtain a sliding of the mobile robotic apparatus (1) along obstacles encountered within the space to be disinfected along the path trajectory taken.

3. The method (600) according to claim 1 or 2, comprising a step of:

   - h) performing (608) a procedure for optimizing a plurality of parameters used to determine the respective virtual potential of attraction of the at least one UV-C radiation source (4) towards each contact surface to be disinfected of said plurality of contact surfaces to be disinfected, based on different values of virtual potential of attraction of the at least one UV-C radiation source (4) towards each contact surface to be disinfected of said plurality of contact surfaces to be disinfected, said optimized parameters being representative of path trajectories (TP) capable of ensuring that the mobile robotic apparatus (1) travels through the space (100) to be disinfected in an set minimum time, providing an appropriate radiation dose on each contact surface to be disinfected.

4. The method (600) according to any one of the preceding claims, wherein the step of a) acquiring (601) can be directly performed by the data processing unit (5) of the mobile robotic apparatus (1) or is provided by a remote electronic computer (20) with respect to the mobile robotic apparatus (1) and in communication therewith by means of a data communication network.

5. The method (600) according to any one of the preceding claims, wherein the step of b) acquiring (602) can be directly performed by the data processing unit (5) of the mobile robotic apparatus (1) or is provided by a remote electronic computer (20) with respect to the mobile robotic apparatus (1) and in communication therewith by means of a data communication network.

6. The method (600) according to any one of the claims, wherein the set operating features of the at least one UV-C radiation source (4) comprise: surface, geometric arrangement, shape, radiation power.

7. A mobile robotic apparatus (1) for disinfecting a space (100), comprising:

- a mobile base (2) comprising a plurality of wheels (3);
- at least one C-type ultraviolet light radiation source (4) mounted to said mobile base (2);
- a data processing unit (5) operatively connected to the mobile base (2) and to the at least one C-type ultraviolet light radiation source (4),
- at least one first artificial vision sensor (7) operatively connected to the data processing unit (5);

said data processing unit (5) being configured to perform a control method according to any one of the preceding claims.

8. The mobile robotic apparatus (1) according to claim 7, comprising a plurality of odometry sensors (3'), each odometry sensor of said plurality of odometry sensors (3') being operatively associated with a wheel of said plurality of wheels (3), the plurality of odometry sensors (3') being operatively connected to the data processing unit (5).

9. The mobile robotic apparatus (1) according to any one of the preceding claims 7 and 8, further comprising at least one second artificial vision sensor (8) operatively connected to the data processing unit (5).

10. The mobile robotic apparatus according to any one of the preceding claims 7 to 9, further comprising a data communication module (17) operatively connected to the data processing unit (5) of the mobile robotic apparatus (1), the data communication module (17) being configured to be operatively connected to a remote computer (20) of a user by means of a data communication network.

**Patentansprüche**

1. Verfahren (600) zur Steuerung einer mobilen Robotervorrichtung (1) zur Desinfektion eines Raumes (100), umfassend folgende Schritte:

a) Erfassen (601), durch eine Datenverarbeitungseinheit (5) einer mobilen Robotervorrichtung (1) zur Desinfektion eines Raumes (100), einer Karte (M) eines zu desinfizierenden Raumes (100);
b) Erfassen (602), durch die Datenverarbeitungseinheit (5) der mobilen Vorrichtung (1), Informationen über eine Vielzahl von zu desinfizierenden Kontaktflächen innerhalb des zu desinfizierenden Raumes (100), wobei jede zu desinfizierende Kontaktfläche einem Kritikalitätsgrad zugeordnet ist, in Bezug darauf ein entsprechender Desinfektionsgrad gewährleistet werden muss;
c) Bestimmen (603), durch die Datenverarbeitungseinheit (5) der mobilen Robotervorrichtung (1), einer Menge an UV Licht-Strahlungsenergie des C-Typs, die mittels mindestens einer UV Licht-C, UV-C, Strahlungsquelle (4), die an der mobilen Robotervorrichtung (1) eingebaut wird, auf eine jeweilige zu desinfizierende Kontaktfläche der Vielzahl von zu desinfizierenden Kontaktflächen abgesetzt werden soll, wobei die Menge der UV-C-Strahlungsenergie in Abhängigkeit von dem Kritikalitätsgrad der zu desinfizierenden Kontaktfläche, dem Abstand und der Orientierung der zu desinfizierenden Kontaktfläche in Bezug auf mindestens eine UV-C-Strahlungsquelle (4) sowie den eingestellten Betriebsmerkmalen der mindestens einen UV-C-Strahlungsquelle (4) bestimmt wird;
d) Bestimmen (604), zu jedem Zeitpunkt $t_i$ 1 < i < N, einer Vielzahl von Zeitpunkten, durch die Datenverarbeitungseinheit (5) der mobilen Robotervorrichtung (1), eines jeweiligen virtuellen Anziehungspotentials der mindestens einen UV-C-Strahlungsquelle (4) zu jeder zu desinfizierenden Kontaktfläche der Vielzahl von zu desinfizierenden Kontaktflächen, durch Modulieren im Laufe der Zeit des virtuellen Anziehungspotentials der mindestens einen UV-C-Strahlungsquelle (4) zu jeder zu desinfizierenden Kontaktfläche der Vielzahl von Kontaktflächen in Abhängigkeit von der Menge von Strahlungsenergie, die für jede zu desinfizierende Kontaktfläche bestimmt wird und in dem vorhergehenden Zeitpunkt $t_{i-1}$ bestrahlt wird, wobei das virtuelle Anziehungspotential ein Wert des Energieniveau ist, der durch ein mathematisches Modell, das ein Anziehungs- oder Abstoßungsgesetz zu jeder zu desinfizierenden Kontaktfläche der Vielzahl von desinfizierenden Kontaktflächen darstellt, bestimmbar ist;

e) Erzeugen (605), durch die Datenverarbeitungseinheit (5) der mobilen Robotervorrichtung (1), einer Pfad-Trajektorie (TP) in Abhängigkeit jedes bestimmten virtuellen Anziehungspotentials der mindestens einen UV-C-Strahlungsquelle (4) zu jeder zu desinfizierenden Kontaktfläche, wobei die Pfad-Trajektorie als Richtungsgeschwindigkeit oder Beschleunigung erzeugt wird, die der mobilen Robotervorrichtung (1) entweder in Echtzeit oder in Simulation bereitgestellt werden soll, basierend auf jedem bestimmten virtuellen Anziehungspotential der mindestens einen UV-C-Strahlungsquelle (4) zu jeder zu desinfizierenden Kontaktfläche, umfassend auch die Energie, die von jeder zu desinfizierenden Kontaktfläche in dem vorherigen Durchfahrt der mobilen Robotervorrichtung (1) gespeichert wird;

f) Steuern (606), durch die Datenverarbeitungseinheit (5) der mobilen Robotervorrichtung (1), der mobilen Robotervorrichtung (1) entlang der erzeugten Pfad-Trajektorie (TP).

2. Verfahren (600) nach Anspruch 1, wobei Schritt e) ferner einen Schritt umfasst:

g) Erzeugen (607) der Pfad-Trajektorie (TP) basierend auf einem Gradienten des bestimmten virtuellen Anziehungspotentials der mindestens einen UV-C-Strahlungsquelle (4) zu jeder Kontaktfläche, um ein Gleiten der mobilen Robotervorrichtung (1) entlang von Hindernissen, die in dem zu desinfizierenden Raum entlang der zurückgelegten Trajektorie angetroffenen werden, zu erhalten.

3. Verfahren (600) nach Anspruch 1 oder 2, umfassend einen Schritt von:

h) Ausführen (608) eines Verfahrens zur Optimierung einer Vielzahl von Parametern, die verwendet werden, um das jeweilige virtuelle Anziehungspotential der mindestens einen UV-C-Strahlungsquelle (4) zu jeder zu desinfizierenden Kontaktfläche der Vielzahl von zu desinfizierenden Kontaktflächen basierend auf den unterschiedlichen Werten des virtuellen Anziehungspotentials der mindestens einen UV-C-Strahlungsquelle (4) zu jeder zu desinfizierenden Kontaktfläche der Vielzahl von zu desinfizierenden Kontaktflächen zu bestimmen, wobei die optimierten Parameter für Pfad-Trajektorien (TP) darstellend sind, die in der Lage sind, zu gewährleisten, dass die mobile Robotervorrichtung (1) den zu desinfizierenden Raum (100) in einer eingestellten Mindestzeit durchläuft und eine geeignete Strahlendosis auf jede zu desinfizierende Kontaktfläche bereitstellt.

4. Verfahren (600) nach einem der vorhergehenden Ansprüche, wobei der Schritt a) von Erfassen (601) direkt durch die Datenverarbeitungseinheit (5) der mobilen Robotervorrichtung (1) durchgeführt werden kann oder von einem elektronischen Computer (20), der in Bezug auf die mobile Robotervorrichtung (1) entfernt ist, bereitgestellt wird, der mit dieser über ein Datenkommunikationsnetzwerk kommuniziert.

5. Verfahren (600) nach einem der vorhergehenden Ansprüche, wobei der Schritt b) von Erfassen (602) direkt durch die Datenverarbeitungseinheit (5) der mobilen Robotervorrichtung (1) durchgeführt werden kann oder von einem elektronischen Computer (20), der in Bezug auf die mobile Robotervorrichtung (1) entfernt ist, bereitgestellt wird, der mit dieser über ein Datenkommunikationsnetzwerk kommuniziert.

6. Verfahren (600) nach einem der vorhergehenden Ansprüche, wobei die eingestellten Betriebsmerkmale der mindestens einen UV-C-Strahlungsquelle (4) umfassen: Oberfläche, geometrische Anordnung, Form, Strahlungsleistung.

7. Mobile Robotervorrichtung (1) zur Desinfektion eines Raumes (100), umfassend:

   - eine mobile Basis (2) umfassend eine Vielzahl von Rädern (3);
   - mindestens eine UV-Strahlungsquelle (4) des Typs C, die an der mobilen Basis (2) eingebaut wird;
   - eine Datenverarbeitungseinheit (5), die operativ mit der mobilen Basis (2) und mit der mindestens einen UV-Strahlungsquelle (4) des Typs C verbunden ist,
   - mindestens einen ersten künstlichen Vision-Sensor (7), der operativ mit der Datenverarbeitungseinheit (5) verbunden ist;

wobei die Datenverarbeitungseinheit (5) dazu konfiguriert ist, ein Steuerverfahren nach einem der vorhergehenden Ansprüche auszuführen.

8. Mobile Robotervorrichtung (1) nach Anspruch 7, umfassend eine Vielzahl von Odometriesensoren (3'), wobei jeder Odometriesensor der Vielzahl von Odometriesensoren (3') operativ einem Rad der Vielzahl von Rädern (3) zugeordnet ist, wobei die Vielzahl von Odometriesensoren (3') operativ mit der Datenverarbeitungseinheit (5) verbunden ist.

9. Mobile Robotervorrichtung (1) nach einem der vorhergehenden Ansprüche 7 und 8, ferner umfassend mindestens einen zweiten künstlichen Vision-Sensor (8), der operativ mit der Datenverarbeitungseinheit (5) verbunden ist.

10. Mobile Robotervorrichtung nach einem der vorhergehenden Ansprüche 7 bis 9, ferner umfassend ein Datenkommunikationsmodul (17), das operativ mit der Datenverarbeitungseinheit (5) der mobilen Robotervorrichtung (1) verbunden ist, wobei das Datenkommunikationsmodul (17) dazu konfiguriert ist, operativ mit einem entfernten Computer (20) eines Benutzers über ein Datenkommunikationsnetzwerk verbunden zu werden.

**Revendications**

1. Procédé (600) de commande d'un appareil robotique mobile (1) pour désinfecter un espace (100), comprenant des étapes consistant à :

   a) acquérir (601), par une unité de traitement de données (5) d'un appareil robotique mobile (1) pour désinfecter un espace (100), une carte (M) d'un espace (100) à désinfecter ;
   b) acquérir (602), par l'unité de traitement de données (5) de l'appareil robotique mobile (1), des informations sur une pluralité de surfaces de contact à désinfecter à l'intérieur de l'espace (100) à désinfecter, chaque surface de contact à désinfecter étant associée à un niveau de criticité par rapport auquel un niveau de désinfection correspondant doit être garanti ;
   c) déterminer (603), par l'unité de traitement de données (5) de l'appareil robotique mobile (1), une quantité d'énergie de rayonnement ultraviolet de type C à déposer, au moyen d'au moins une source de rayonnement ultraviolet-C, UV-C, (4) montée sur l'appareil robotique mobile (1), sur une surface de contact respective à désinfecter de ladite pluralité de surfaces de contact à désinfecter, ladite quantité d'énergie de rayonnement UV-C étant déterminée en fonction du niveau de criticité de la surface de contact à désinfecter, de la distance et de l'orientation de la surface de contact à désinfecter par rapport à l'au moins une source de rayonnement UV-C (4), des caractéristiques de fonctionnement définies de l'au moins une source de rayonnement UV-C (4) ;
   d) déterminer (604), pour chaque instant $t_i$, $1 < i < N$, d'une pluralité d'instants, par l'unité de traitement de données (5) de l'appareil robotique mobile (1), un potentiel virtuel respectif d'attraction de l'au moins une source de rayonnement UV-C (4) vers chaque surface de contact à désinfecter de ladite pluralité de surfaces de contact à désinfecter, en modulant dans le temps le potentiel virtuel d'attraction de l'au moins une source de rayonnement UV-C (4) vers chaque surface de contact à désinfecter de ladite pluralité de surfaces de contact en fonction de la quantité d'énergie de rayonnement déterminée pour chaque surface de contact à désinfecter et irradiée à l'instant précédent $t_{i-1}$, ledit potentiel virtuel d'attraction étant une valeur de niveau d'énergie déterminable au moyen d'un modèle mathématique représentatif d'une loi d'attraction ou de répulsion vers chaque surface de contact à désinfecter de ladite pluralité de surfaces de contact à désinfecter ;
   e) générer (605), par l'unité de traitement de données (5) de l'appareil robotique mobile (1), une trajectoire de parcours (TP) en fonction de chaque potentiel virtuel d'attraction déterminé de l'au moins une source de rayonnement UV-C (4) vers chaque surface de contact à désinfecter, la trajectoire de parcours étant générée comme vitesse ou accélération directionnelle à fournir à l'appareil robotique mobile (1), en temps réel ou en simulation, sur la base de chaque potentiel virtuel d'attraction déterminé de l'au moins une source de rayonnement UV-C (4) vers chaque surface de contact à désinfecter, comprenant également l'énergie stockée par chaque surface de contact à désinfecter lors du passage précédent de l'appareil robotique mobile (1) ;
   f) commander (606), par l'unité de traitement de données (5) de l'appareil robotique mobile (1), l'appareil robotique mobile (1) le long de la trajectoire de parcours générée (TP).

2. Procédé (600) selon la revendication 1, dans lequel l'étape e) comprend en outre une étape consistant à :
   g) générer (607) la trajectoire de parcours (TP) sur la base d'un gradient du potentiel virtuel d'attraction déterminé de l'au moins une source de rayonnement UV-C (4) vers chaque surface de contact afin d'obtenir un glissement de l'appareil robotique mobile (1) le long des obstacles rencontrés dans l'espace à désinfecter le long de la trajectoire de parcours suivie.

3. Procédé (600) selon la revendication 1 ou 2, comprenant une étape consistant à :
   h) exécuter (608) une procédure d'optimisation d'une pluralité de paramètres utilisés pour déterminer le potentiel virtuel respectif d'attraction de l'au moins une source de rayonnement UV-C (4) vers chaque surface de contact à désinfecter de ladite pluralité de surfaces de contact à désinfecter, sur la base de différentes valeurs de potentiel virtuel d'attraction de l'au moins une source de rayonnement UV-C (4) vers chaque surface de contact à désinfecter de ladite pluralité de surfaces de contact à désinfecter, lesdits paramètres optimisés étant représentatifs de trajectoires de parcours (TP) capables de garantir que l'appareil robotique mobile (1) parcourt l'espace (100) à désinfecter dans un temps minimal défini, en fournissant une dose de rayonnement appropriée sur chaque surface de contact à désinfecter.

4. Procédé (600) selon l'une quelconque des revendications précédentes, dans lequel l'étape a) d'acquisition (601) peut être directement exécutée par l'unité de traitement de données (5) de l'appareil robotique mobile (1) ou est fournie par un ordinateur électronique distant (20) par rapport à l'appareil robotique mobile (1) et en communication avec celui-ci au moyen d'un réseau de communication de données.

5. Procédé (600) selon l'une quelconque des revendications précédentes, dans lequel l'étape b) d'acquisition (602) peut être directement exécutée par l'unité de traitement de données (5) de l'appareil robotique mobile (1) ou est fournie par un ordinateur électronique distant (20) par rapport à l'appareil robotique mobile (1) et en communication avec celui-ci au moyen d'un réseau de communication de données.

6. Procédé (600) selon l'une quelconque des revendications, dans lequel les caractéristiques de fonctionnement définies de l'au moins une source de rayonnement UV-C (4) comprennent : surface, configuration géométrique, forme, puissance de rayonnement.

7. Appareil robotique mobile (1) pour désinfecter un espace (100), comprenant :

   une base mobile (2) comprenant une pluralité de roues (3) ;
   au moins une source de rayonnement ultraviolet de type C (4) montée sur ladite base mobile (2) ;
   une unité de traitement de données (5) connectée fonctionnellement à la base mobile (2) et à l'au moins une source de rayonnement ultraviolet de type C (4),
   au moins un premier capteur de vision artificielle (7) connecté fonctionnellement à l'unité de traitement de données (5) ;
   ladite unité de traitement de données (5) étant configurée pour exécuter un procédé de commande selon l'une quelconque des revendications précédentes.

8. Appareil robotique mobile (1) selon la revendication 7, comprenant une pluralité de capteurs d'odométrie (3'), chaque capteur d'odométrie de ladite pluralité de capteurs d'odométrie (3') étant associé fonctionnellement à une roue de ladite pluralité de roues (3), la pluralité de capteurs d'odométrie (3') étant connectée fonctionnellement à l'unité de traitement de données (5).

9. Appareil robotique mobile (1) selon l'une quelconque des revendications précédentes 7 et 8, comprenant en outre au moins un second capteur de vision artificielle (8) connecté fonctionnellement à l'unité de traitement de données (5).

10. Appareil robotique mobile selon l'une quelconque des revendications précédentes 7 à 9, comprenant en outre un module de communication de données (17) connecté fonctionnellement à l'unité de traitement de données (5) de l'appareil robotique mobile (1), le module de communication de données (17) étant configuré pour être connecté fonctionnellement à un ordinateur distant (20) d'un utilisateur au moyen d'un réseau de communication de données.

Fig. 1a

Fig. 1b

Fig. 2

Fig. 3

Fig. 4b

Fig. 4a

Fig. 5b

Fig. 5a

Fig. 5c

Fig. 5d

EP 4 052 733 B1

Fig. 5e

Fig. 5f

EP 4 052 733 B1

Fig. 6

**EP 4 052 733 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016077403 A1

- EP 3033115 A1